# EUROPEAN PATENT APPLICATION

(11) **EP 2 263 662 A1**
(43) Date of publication of application: **22.12.2010**
(21) Application number: 09732215.0
(22) Date of filing: 13.04.2009
(51) Int. Cl.: A61K 9/70, A23L 1/00, A61K 47/02, A61K 47/10, A61K 47/26, A61K 47/32, A61K 47/34, A61K 47/36, A61K 47/38

(54) **FILM-LIKE COMPOSITION**

(30) Priority: 15.04.2008 JP 2008106142
(71) Applicant: Shionogi&Co., Ltd., Osaka-shi Osaka 541-0045 (JP)
(72) Inventor: FUNAKI, Takeshi, Amagasaki-shi Hyogo 660-0813 (JP); TAKASE, Yuriko, Amagasaki-shi Hyogo 660-0813 (JP)
(74) Representative: Teipel, Stephan
(86) International application number: PCT/JP2009/057461
(87) International publication number: WO 2009/128433

(57) **Abstract**

A film composition comprising (i) a resin obtained by copolymerizing polyvinyl alcohol and at least one or more polymerizable vinyl monomers; and (ii) a drug or a food component.

## Description

### TECHNICAL FIELD

The present invention relates to a novel film composition, and in particular to a film composition comprising a polyvinyl alcohol copolymer and either a drug or a food component.

### BACKGROUND ART

Various kinds of drug-containing films that rapidly dissolve in the mouth have so far been developed so that drugs are taken orally without any problems associated with swallowing and the like.

For example, JP-A-4-266819 discloses a drug-containing film obtained using, as a base, a vinylpyrrolidone homopolymer and a methacrylic acid-methyl methacrylate copolymer at a weight ratio of 99:1 to 50:50. This drug-containing film is not entirely satisfactory in respect of its drug content and its usability (disintegrability, and the like). JP-A-9-235220 also discloses a multi-layer drug-containing film comprising three layers of an adhesive layer, an intermediate layer (drug-containing layer), and a poorly water-soluble layer (non-adhesive layer). Because of the multi-layer structure, the production process of this drug-containing film is complicated.

In order to solve the above problems, a use of hydroxypropyl cellulose, hydroxypropyl methyl cellulose and/or the like as a base, together with sugars (for example, maltitol syrup, or the like) to obtain a drug-containing film (JP-A-11-116469); and a use of either methyl cellulose or hydroxypropyl methyl cellulose as a base, without sugars to obtain a drug-containing film (JP-A-2005-232072) have been suggested. These films, however, are disadvantageous when the drug contents thereof are large because the films cannot be formed and because their disintegration time in the mouth extends and thereby affects ease of ingestion.

Meanwhile a resin obtained by copolymerizing polyvinyl alcohol and at least one or more polymerizable vinyl monomers (hereinafter a polyvinyl alcohol copolymer) has been used for a base of a film coating for tablets (WO 2005/19286), a coated layer of an ink jet sheet (JP-A-62-288076), and a film coating for hard capsules (WO 02/17848). However, each film in the above WO 2005/19286, JP-A-62-288076, and WO 02/17848 does not contain any drugs or food components and is not a film composition for a drug or a food component.

### SUMMARY OF INVENTION

### TECHNICAL PROBLEM

An object of the present invention is to provide a film composition that is easily formed and contains a drug or a food component.

The object of the present invention is more preferably to provide a film composition (for example, used as a pharmaceutical preparation, or a food) that is easily formed even when the film composition contains a large amount of a drug or a food component. Unlike conventional drug-containing films, the film composition of the present invention rapidly disintegrates in the mouth and can be produced by a simple process.

### SOLUTION TO PROBLEM

The inventors of the present invention conducted extensive investigations to solve the problems mentioned above, and found that with the use of a resin obtained by copolymerizing polyvinyl alcohol and at least one or more polymerizable vinyl monomers (a polyvinyl alcohol copolymer) as a base of a film composition, a drug-containing film or a food-containing film that is easily formed and taken orally can be produced even when the film contains a large amount of a drug or a food component. The present invention was thus completed.

That is, the present invention relates to
(1) a film composition comprising
   (i) a resin obtained by copolymerizing polyvinyl alcohol and at least one or more polymerizable vinyl monomers; and
   (ii) a drug or a food component;
(2) the composition according to (1), wherein the composition is a drug-containing film comprising the resin obtained by copolymerizing the polyvinyl alcohol and the at least one or more polymerizable vinyl monomers; and the drug;
(3) the composition according to (1), wherein the composition is a food-containing film comprising the resin obtained by copolymerizing the polyvinyl alcohol and the at least one or more polymerizable vinyl monomers; and the food component;
(4) the composition according to any of (1) to (3), wherein the polyvinyl alcohol and the polymerizable vinyl monomer are copolymerized at a weight ratio of 6:4 to 9:1;
(5) the composition according to any of (1) to (4), wherein the polyvinyl alcohol has an average degree of polymerization of 1300 or less;
(6) the composition according to (5), wherein the polyvinyl alcohol has an average degree of polymerization of 200 to 1300;
(7) the composition according to (5), wherein the polyvinyl alcohol has an average degree of polymerization of 200 to 900;
(8) the composition according to (5), wherein the polyvinyl alcohol has an average degree of polymerization of 300 to 500;
(9) the composition according to any of (1) to (8), wherein the polyvinyl alcohol is partially hydrolyzed polyvinyl alcohol;
(10) the composition according to any of (1) to (9), wherein the polymerizable vinyl monomer is at least one or more selected from the group consisting of acrylic acid, methacrylic acid, methyl acrylate, ethyl acrylate, butyl acrylate, isobutyl acrylate, methyl methacrylate, ethyl methacrylate, butyl methacrylate, and isobutyl methacrylate;
(11) the composition according to any of (1) to (10), wherein the resin is a copolymer of partially hydrolyzed polyvinyl alcohol having an average degree of polymerization of 300 to 500, methyl methacrylate, and acrylic acid;
(12) the composition according to (11), wherein the resin is a copolymer obtained by copolymerizing partially hydrolyzed polyvinyl alcohol having an average degree of polymerization of 300 to 500, methyl methacrylate, and acrylic acid at a weight ratio of 60 to 90:7 to 38:0.5 to 12;
(13) the composition according to any of (1) to (12), wherein the amount of the resin is 1 to 90% by weight relative to the total weight of the composition;
(14) the composition according to (13), wherein the amount of the resin is 60 to 80% by weight relative to the total weight of the composition;
(15) the composition according to any of (1) to (14), wherein the amount of the drug or the food component is 0.01 to 50% by weight relative to the total weight of the composition;
(16) the composition according to (15), wherein the amount of the drug or the food component is 5 to 30% by weight relative to the total weight of the composition;
(17) the composition according to any of (1) to (16), wherein the composition further comprises at least one or more selected from the group consisting of a disintegrant, an excipient, and a binder;
(18) the composition according to (17), wherein the disintegrant is at least one or more selected from the group consisting of crospovidone, starch, carmellose, carmellose calcium, carboxy starch sodium, carboxymethyl starch sodium, low-substituted hydroxypropyl cellulose, guar gum, croscarmellose sodium, pregelatinized starch, agar powder, and gum arabic;
(19) the composition according to (18), wherein the disintegrant is crospovidone;
(20) the composition according to (18) or (19), wherein the amount of the disintegrant is 1 to 50% by weight relative to the total weight of the composition;
(21) the composition according to (20), wherein the amount of the disintegrant is 5 to 20% by weight relative to the total weight of the composition;
(22) the composition according to (17), wherein the excipient is at least one or more selected from the group consisting of maltitol syrup, crystalline cellulose, erythritol, D-sorbitol, xylitol, sorbitol, trehalose, D-mannitol, maltitol, mannitol, glucose, fructose, maltose, sucrose, starch syrup, saccharose, glucose, lactose, anhydrous silicic acid, dibasic calcium phosphate, anhydrous dibasic calcium phosphate, calcium silicate, hydrous silicon dioxide, magnesium aluminometasilicate, synthetic hydrotalcite, calcium carbonate, precipitated calcium carbonate, magnesium carbonate, corn starch, potato starch, wheat starch, rice starch, and partially pregelatinized starch;
(23) the composition according to (22), wherein the excipient is maltitol syrup;
(24) the composition according to (22) or (23), wherein the amount of the excipient is 1 to 50% by weight relative to the total weight of the composition;
(25) the composition according to (24), wherein the amount of the excipient is 5 to 20% by weight relative to the total weight of the composition;
(26) the composition according to (17), wherein the binder is at least one or more selected from the group consisting of hydroxypropyl methyl cellulose, hydroxypropyl cellulose, polyvinylpyrrolidone, methyl cellulose, polyvinyl acetal diethylamino acetate, a hydroxypropyl methyl cellulose mixture, carmellose, carmellose sodium, carboxy starch sodium, croscarmellose sodium, crospovidone, hydroxyethyl methyl cellulose, hydroxypropyl starch, hydroxyethyl starch, crystalline cellulose, pullulan, gum arabic, and sodium alginate;
(27) the composition according to any of (17) to (26), wherein the composition comprises, relative to the total weight of the composition, 1 to 90% by weight of the resin that is a copolymer obtained by copolymerizing partially hydrolyzed polyvinyl alcohol having an average degree of polymerization of 300 to 500, methyl methacrylate, and acrylic acid at a weight ratio of 60 to 90:7 to 38:0.5 to 12; 0.01 to 50% by weight of the drug or the food component; 1 to 50% by weight of the disintegrant; and 1 to 50% by weight of the excipient; and
(28) the composition according to (27), wherein the composition comprises, relative to the total weight of the composition, 60 to 80% by weight of the resin that is a copolymer obtained by copolymerizing partially hydrolyzed polyvinyl alcohol having an average degree of polymerization of 300 to 500, methyl methacrylate, and acrylic acid at a weight ratio of 60 to 90:7 to 38:0.5 to 12; 5 to 30% by weight of the drug or the food component; 5 to 20% by weight of the disintegrant; and 5 to 20% by weight of the excipient.

### ADVANTAGEOUS EFFECTS OF INVENTION

With the use of the polyvinyl alcohol copolymer mentioned above as a base of a film composition, the present invention can provide a stable film composition that is preferably easily formed even when the composition contains a large amount of a drug or a food component. The film composition of the present invention preferably has excellent film strength and a short dissolution time in the mouth.

### DESCRIPTION OF EMBODIMENTS

The film composition of the present invention widely refers to a film composition for delivering its main component into the body of an animal including a human. When the main component is a drug, the composition of the present invention is a pharmaceutical preparation. When the main component is a food component, the composition of the present invention is a food.

The polyvinyl alcohol copolymer used as a base in the present invention can be produced by copolymerizing polyvinyl alcohol and at least one or more polymerizable vinyl monomers through a method known per se.

The polyvinyl alcohol and the at least one or more polymerizable vinyl monomers are preferably copolymerized at a weight ratio of about 6:4 to 9:1 (the polyvinyl alcohol: the polymerizable vinyl monomers).

Polyvinyl alcohol having an average degree of polymerization of 1300 or less and the at least one or more polymerizable vinyl monomers are preferably copolymerized at a weight ratio of about 6: 4 to 9:1 (the polyvinyl alcohol having an average degree of polymerization of 1300 or less: the polymerizable vinyl monomers).

Polyvinyl alcohol having an average degree of polymerization of 200 to 1300 and the at least one or more polymerizable vinyl monomers are preferably copolymerized at a weight ratio of about 6: 4 to 9:1 (the polyvinyl alcohol having an average degree of polymerization of 200 to 1300: the polymerizable vinyl monomers).

Examples of a method for producing such a polyvinyl alcohol copolymer include a method described in WO 2005/019286. Specific examples of the method include radical polymerization such as solution polymerization, suspension polymerization, emulsion polymerization, and bulk polymerization. Each of the methods is known per se and can be carried out under a usual polymerization condition. The polymerization reaction is usually carried out in water, an organic solvent (for example, methanol, ethanol, cellosolve, carbitol, and the like), or a mixture thereof in the presence of a polymerization initiator, and if necessary in the presence of a reducing agent (for example, sodium erythorbate, sodium metabisulfite, ascorbic acid, and the like); a chain transfer agent (for example, 2-mercaptoethanol, α-methylstyrene dimer, 2-ethylhexyl thioglycolate, lauryl mercaptan, and the like); and/or a dispersing agent (for example, a surfactant such as sorbitan ester, lauryl alcohol, and the like). A removing method of an unreacted monomer, a drying method, a crushing method, and the like are also carried out by a known method, and not specifically limited.

The polyvinyl alcohol used as a raw material of the polyvinyl alcohol copolymer of the present invention has an average degree of polymerization of about 200 to 1300, preferably about 200 to 900, more preferably about 300 to 500. The polyvinyl alcohol may also be partially hydrolyzed polyvinyl alcohol having a degree of hydrolysis of about 60 mol% or more, preferably about 78 to 96 mol%. Such hydrolyzed polyvinyl alcohol can be produced by radically polymerizing vinyl acetate and subsequently suitably hydrolyzing the obtained vinyl acetate. A desired polyvinyl alcohol can be produced by suitably controlling the degree of polymerization and hydrolysis by a method known per se.

Alternatively, the partially hydrolyzed polyvinyl alcohol may be a marketed product. Preferred examples of the marketed product of the polyvinyl alcohol include GOHSENOL EG-05(NIPPON GOHSEI Co., Ltd.), GOHSENOL EG-25 (NIPPON GOHSEI Co., Ltd.), PVA203 (Kuraray Co., Ltd.), PVA204 (Kuraray Co. , Ltd.), PVA205 (Kuraray Co., Ltd.), JP-04 (JAPAN VAM & POVAL Co., Ltd.), and JP-05 (JAPAN VAM & POVAL Co., Ltd.). When one kind of polyvinyl alcohol alone is used as a raw material in the production of the polyvinyl alcohol copolymer, which is a main component of the composition of the present invention, the polyvinyl alcohol has an average degree of polymerization of 1300 or less, preferably about 200 to 1300, more preferably about 200 to 900, especially preferably about 300 to 500. In the production of the polyvinyl alcohol copolymer, which is a main component of the composition of the present invention, instead of using one kind of polyvinyl alcohol, a combination of two or more kinds of polyvinyl alcohols having different degrees of polymerization and/or hydrolysis may also be used as a raw material depending on the purpose. When the combination of two or more kinds of polyvinyl alcohols having different degrees of polymerization and/or hydrolysis is used depending on the purpose, a mixture of polyvinyl alcohol having an average degree of polymerization of 200 to 500 and polyvinyl alcohol having an average degree of polymerization of 1000 to 1500 may be used. A marketed premixed coating agent containing polyvinyl alcohol may also be used.

The polyvinyl alcohol used as a raw material in the present invention may be various kinds of modified polyvinyl alcohols. Examples of the modified polyvinyl alcohols include amine-modified polyvinyl alcohol, ethylene-modified polyvinyl alcohol, carboxylic acid-modified polyvinyl alcohol, diacetone-modified polyvinyl alcohol, and thiol-modified polyvinyl alcohol. Such a modified polyvinyl alcohol may be a marketed product or a modified polyvinyl alcohol produced by a method known in the art.

Examples of the polymerizable vinyl monomer to be polymerized with the polyvinyl alcohol as a raw material include unsaturated carboxylic acids such as acrylic acid, methacrylic acid, crotonic acid, fumaric acid, maleic acid, and itaconic acid; esters of unsaturated carboxylic acids (for example, a substituted or unsubstituted alkyl ester, a cyclic alkyl ester, a polyalkylene glycol ester, and the like); unsaturated nitriles; unsaturated amides; aromatic vinyls; aliphatic vinyls; unsaturated bond-containing heterocyclic compounds; and salts thereof (for example, an alkali metal salt, an ammonium salt, an alkylamine salt, and the like). Among these, unsaturated carboxylic acids and esters of unsaturated carboxylic acids are preferable. Specific examples whereof include (1) acrylic acid esters such as methyl acrylate, ethyl acrylate, butyl acrylate, isobutyl acrylate, cyclohexyl acrylate, 2-ethylhexyl acrylate, hydroxyethyl acrylate, polyethylene glycol acrylate (an ester of polyethylene glycol and acrylic acid), and polypropylene glycol acrylate (an ester of polypropylene glycol and acrylic acid); (2) methacrylic acid esters such as methyl methacrylate, ethyl methacrylate, butyl methacrylate, isobutyl methacrylate, cyclohexyl methacrylate, 2-ethylhexyl methacrylate, hydroxyethyl methacrylate, and polyethylene glycol methacrylate (an ester of polyethylene glycol and methacrylic acid); (3) unsaturated nitriles such as acrylonitrile, and methacrylonitrile; (4) unsaturated amides such as acrylamide, dimethylacrylamide, and methacrylamide; (5) aromatic vinyls such as styrene, and α-methyl styrene; (6) aliphatic vinyls such as vinyl acetate; and (7) unsaturated bond-containing heterocyclic compounds such as N-vinylpyrrolidone, and acryloyl morpholine. Among these, it is preferable to use acrylic acid, methacrylic acid, methyl acrylate, ethyl acrylate, butyl acrylate, isobutyl acrylate, methyl methacrylate, ethyl methacrylate, butyl methacrylate, and/or isobutyl methacrylate.

The polymerizable vinyl monomer alone, or in combination of two or more, can be copolymerized with the polyvinyl alcohol. Regarding a preferable combination, it is preferable to copolymerize the polyvinyl alcohol with a mixture of acrylic acid and a methacrylic acid ester (for example, methyl methacrylate, or the like). The polyvinyl alcohol and the polymerizable vinyl monomers are copolymerized at a weight ratio of, for example, about 6: 4 to about 9: 1, preferably about 7.5:2.5 to about 8.5:1.5, especially preferably about 8:2. When acrylic acid and methyl methacrylate are used as the polymerizable vinyl monomers, the weight ratio thereof is, for example, about 3:7 to about 0.5:9.5, preferably about 2:8 to about 1:9, especially preferably about 1.3:8.7. A preferable polyvinyl alcohol copolymer used as a main component of the coating composition of the present invention is a copolymer of polyvinyl alcohol (the average polymerization degree of about 200 to 1300), methyl methacrylate, and acrylic acid, and the weight ratio of each component (the polyvinyl alcohol having an average degree of polymerization of about 200 to 1300: the methyl methacrylate: the acrylic acid) is about 60 to 90:7 to 38:0.5 to 12, especially preferably about 80:17.5:2.5.

The weight ratio of polyvinyl alcohol, methyl methacrylate, and acrylic acid used in copolymerization is about 60 to 90:7 to 38:0.5 to 12, which is the same as the weight ratio of polyvinyl alcohol, methyl methacrylate, and acrylic acid in a resulting copolymer. The weight ratio can be determined by nuclear magnetic resonance (NMR).

As the polymerization initiator, a polymerization initiator used in the art can be used. Examples of the polymerization initiator include an inorganic peroxide such as potassium persulfate, ammonium persulfate, and hydrogen peroxide; an organic peroxide such as peracetic acid, tertiary butyl hydroperoxide, and di-n-propyl peroxydicarbonate; an azo compound such as 2,2'-azobis(2-amidinopropane) hydrochloride, and 2,2'-azobis(2,4-dimethylvaleronitrile).

The amount of the resin in the film composition of the present invention is, for example, about 1 to 90% by weight, preferably about 20 to 87.5% by weight, more preferably about 40 to 85% by weight, especially preferably about 60 to 80% by weight, relative to the total weight of the composition. With the use of the resin in these ranges, a drug-containing film or a food-containing film that is easily formed can be produced, and the film can contain a sufficient amount of a drug or a food component.

The drug-containing film of the present invention comprises a drug and the polyvinyl alcohol copolymer. The drug is not specifically limited as long as the drug is a pharmaceutical active ingredient that can be orally or dermally administered. The drug is preferably a drug that can be dissolved or suspended in water and/or an organic solvent. Examples of the organic solvent include an alcohol (for example, ethanol, and methanol), acetone, and methylene chloride. Such an organic solvent may be used alone or in combination of two or more as a mixture. Preferred is ethanol. The term "suspend" in this context refers to making solid particles held in a liquid, the solid particles being in a state of colloidal particles or microscopic particles. Specific examples of the drug include a nutritional tonic, an analgesic-antipyretic-antiinflammatory drug, a psychotropic drug, an anxiolytic, an antidepressant, a sedative-hypnotic drug, a central nervous system drug, a cerebral circulation improver, an antiepileptic, a gastrointestinal drug, an antacid, an antiulcer drug, an antitussive-expectorant drug, an antiemetic, a bronchodilator, an antiallergic, an oral-dental drug, a cardiotonic, an antiarrhythmic, a diuretic, a hypotensive drug, a coronary vasodilator, a peripheral vasodilator, a cholagogue, an antibiotic, a chemotherapeutic drug, an antidiabetic, an osteoporosis drug, a skeletal muscle relaxant, an antispasmodic, an antirheumatic drug, a hormone drug, an alkaloid narcotic, a sulfa drug, an antipodagric, an anticoagulant, and an anticancer drug.

Examples of the nutritional tonic include vitamins such as vitamin A, vitamin D, and vitamin E (acetic acid d-α-tocopherol). Examples of the analgesic-antipyretic-antiinflammatory drug include isopropylantipyrine (IPA), aspirin, acetaminophen, ethenzamide, ibuprofen, noscapine, serrapeptase, lysozyme chloride, tolfenamic acid, mefenamic acid, diclofenac sodium, flufenamic acid, salicylamide, aminopyrine, ketoprofen, indomethacin, bucolome, and pentazocine.

Examples of the psychotropic drug include chlorpromazine, and reserpine. Examples of the anxiolytic include alprazolam, chlordiazepoxide, and diazepam. Examples of the antidepressant include imipramine. Examples of the sedative-hypnotic drug include estazolam, and perlapine. Examples of the central nervous system drug include citicoline. Examples of the cerebral circulation improver include vinpocetine. Examples of the antiepileptic include phenytoin, and carbamazepine. Examples of the gastrointestinal drug include a stomachic and digestive agent such as diastase, saccharated pepsin, scopolia extract, cellulase AP3, lipase AP, and cinnamon oil; and a drug for controlling intestinal functions such as antibiotic-resistant lactic acid bacteria and bifidobacteria.

Examples of the antacid include magnesium carbonate, sodium hydrogen carbonate, magnesium aluminometasilicate, synthetic hydrotalcite, precipitated calcium carbonate, and magnesium oxide. Examples of the antiulcer drug include lansoprazole, omeprazole, rabeprazole, famotidine, cimetidine, and ranitidine hydrochloride. Examples of the antitussive-expectorant drug include theophylline, potassium guaiacolsulfonate, and guaifenesin. Examples of the antiemetic include metoclopramide. Examples of the bronchodilator include theophylline. Examples of the antiallergic include amlexanox, and seratrodast. Examples of the oral-dental drug include oxytetracycline, and triamcinolone acetonide.

Examples of the cardiotonic include digoxin. Examples of the antiarrhythmic include pindolol. Examples of the diuretic include furosemide, and hydrochlorothiazide. Examples of the hypotensive drug include candesartan cilexetil, methyldopa, and perindopril erbumine.

Examples of the coronary vasodilator include molsidomine. Examples of the peripheral vasodilator include cinnarizine. Examples of the cholagogue include trepibutone. Examples of the antibiotic include a cephem antibiotic such as cefadroxil, cefixime, cefditoren pivoxil, cefteram pivoxil, and cefpodoxime proxetil; a synthetic antibacterial agent such as ampicillin, ciclacillin, nalidixic acid, and enoxacin; a monobactam antibiotic such as carumonam sodium; a penem antibiotic; and a carbapenem antibiotic.

Examples of the chemotherapeutic drug include sulfamethizole. Examples of the antidiabetic include tolbutamide, voglibose, glibenclamide, and troglitazone. Examples of the osteoporosis drug include ipriflavone. Examples of the skeletal muscle relaxant include methocarbamol. Examples of the antispasmodic include dimenhydrinate. Examples of the antirheumatic drug include methotrexate, and bucillamine. Examples of the hormone drug include liothyronine sodium, dexamethasone sodium phosphate, prednisolone, oxendolone, and leuprorelin acetate. Examples of the alkaloid narcotic include opium, and ipecacuanha. Examples of the sulfa drug include sulfisomidine, and sulfamethizole. Examples of the antipodagric include allopurinol, and colchicine. Examples of the anticoagulant include dicumarol. Examples of the anticancer drug include 5-fluorouracil, uracil, and mitomycin.

The food-containing film of the present invention comprises a food component and the polyvinyl alcohol copolymer. The food component used for the food-containing film of the present invention is not specifically limited as long as the food component can be ingested as part of the food-containing film. The food component may be in the form of a solid or a liquid at normal temperature.

Examples of the food component include a flavor, a fruit juice, a plant extract, an animal extract, and a vitamin.

Specific examples thereof include menthol, a lemon oil, peppermint, spearmint, a perilla juice, coenzyme Q10, an aloe arborescens extract, a St. John' s wort extract, a milk thistle extract, a ginkgo leaf extract, a vitis vinifera leaf extract, a saw palmetto extract, a pumpkin seed extract, a chaste tree extract, a valerian extract, a hop extract, a rose hip extract, an echinacea extract, a ginger extract, a garlic extract, DHA, EPA, a lactoferrin extract, a vitamin, and an amino acid.

Some of the substances mentioned above, by their nature, serve as both a drug and a food component. A film containing such a substance can be used as a drug-containing film or a food-containing film according to the purpose of the use.

The drug or the food component can be used alone or in combination with any other drug or food component. The amount of the drug or the food component in the film composition of the present invention varies depending on its kind, but the amount is, for example, about 0.01 to 50% by weight, preferably about 0.1 to 40% by weight, more preferably about 1 to 35% by weight, especially preferably about 5 to 30% by weight, relative to the total weight of the film composition. When the amount of the drug or the food component is smaller than these ranges, a large amount of the film composition will have to be taken orally or ingested. On the other hand, when the amount of the drug or the food component is larger than these ranges, the film composition is difficult to form.

The film composition of the present invention can be formed only from the resin mentioned above and either the drug or the food component, but the film composition may further comprise at least one or more selected from the group consisting of a disintegrant, an excipient, and a binder. Some of such additives can simultaneously exhibit the effects of a disintegrant, an excipient, and a binder.

Examples of the disintegrant used in the present invention include crospovidone, starch, carmellose, carmellose calcium, carboxymethyl starch sodium, low-substituted hydroxypropyl cellulose, guar gum, croscarmellose sodium, pregelatinized starch, agar powder, and gum arabic. Among these, crospovidone is especially preferable. The amount of the disintegrant is, for example, about 1 to 50% by weight, preferably about 2 to 40% by weight, more preferably about 3 to 30% by weight, especially preferably about 5 to 20% by weight, relative to the total weight of the film composition. When the amount of the disintegrant is in these ranges, the disintegration time of the film composition in actual use is sufficiently short, and the composition will not disintegrate in the presence of a small amount of water.

Examples of the excipient include sugar alcohols such as maltitol syrup, crystalline cellulose, erythritol, D-sorbitol, xylitol, sorbitol, trehalose, D-mannitol, maltitol, and mannitol; monosaccharides and oligosaccharides, in particular, monosaccharides such as glucose and fructose, oligosaccharides such as maltose and sucrose, starch syrup, and the like; saccharose; glucose; lactose; anhydrous silicic acid; dibasic calcium phosphate; anhydrous dibasic calcium phosphate; calcium silicate; hydrous silicon dioxide; magnesium aluminometasilicate; synthetic hydrotalcite; calcium carbonate; precipitated calcium carbonate; magnesium carbonate; corn starch; potato starch; wheat starch; rice starch; and partially pregelatinized starch. Among these, maltitol syrup is especially preferable. The amount of the excipient is, for example, about 1 to 50% by weight, preferably about 2 to 40% by weight, more preferably about 3 to 30% by weight, especially preferably about 5 to 20% by weight, relative to the total weight of the drug-containing film or the food-containing film. When the amount of the excipient is in these ranges, the film compositions are free from sticking together, and the film composition can contain a sufficient amount of the drug or the food component.

Examples of the binder used in the present invention include hydroxypropyl methyl cellulose (HPMC), hydroxypropyl cellulose (HPC), polyvinylpyrrolidone (PVP), methyl cellulose (MC), polyvinyl acetal diethylamino acetate, a hydroxypropyl methyl cellulose mixture, carmellose, carmellose sodium, carboxy starch sodium, croscarmellose sodium, crospovidone, hydroxyethyl methyl cellulose, hydroxypropyl starch, hydroxyethyl starch, crystalline cellulose, pullulan, gum arabic, and sodium alginate. The amount of the binder is, for example, about 1 to 50% by weight, preferably about 2 to 40% by weight, more preferably about 3 to 30% by weight, especially preferably about 5 to 20% by weight, relative to the total weight of the film composition. When the amount of the binder is in these ranges, the disintegration time of the film composition in actual use is sufficiently short, and the film composition can be easily formed.

Besides the additives mentioned above, other additives known in the art can widely used for the film composition of the present invention, and examples thereof include a plasticizer, a lubricant, a solubilizer, a buffer, a surfactant, in particular, titanium oxide, talc, precipitated calcium carbonate, gelatin, triethyl citrate, triacetin, polyethylene glycol, soybean lecithin, light anhydrous silicic acid, crystalline cellulose, dibasic calcium phosphate, glycerin, lecithin, macrogol, polysorbate 80, sucrose fatty acid ester, sodium lauryl sulfate, and the like.

Preferable embodiments of the film composition of the present invention are as follows. In particular, preferred is a film composition comprising, relative to the total weight of the film composition, 1 to 90% by weight of a resin that is a copolymer obtained by copolymerizing partially hydrolyzed polyvinyl alcohol having an average degree of polymerization of about 300 to 500, methyl methacrylate and acrylic acid at a weight ratio of about 60 to 90:7 to 38:0.5 to 12 (the partially hydrolyzed polyvinyl alcohol having an average degree of polymerization of about 300 to 500: the methyl methacrylate: and the acrylic acid); about 0.01 to 50% by weight of a drug or a food component; about 1 to 50% by weight of a disintegrant; and about 1 to 50% by weight of an excipient. More preferred is a film composition comprising, relative to the total weight of the film composition, about 20 to 87.5% by weight of a resin composition that is a copolymer obtained by copolymerizing partially hydrolyzed polyvinyl alcohol having an average degree of polymerization of about 300 to 500, methyl methacrylate, and acrylic acid at a weight ratio of about 60 to 90:7 to 38:0.5 to 12; about 0.1 to 40% by weight of a drug or a food component; about 2 to 40% by weight of a disintegrant; and about 2 to 40% by weight of an excipient. Further preferred is a film composition comprising, relative to the total weight of the film composition, about 40 to 85% by weight of a resin composition that is a copolymer obtained by copolymerizing partially hydrolyzed polyvinyl alcohol having an average degree of polymerization of about 300 to 500, methyl methacrylate, and acrylic acid at a weight ratio of about 60 to 90:7 to 38:0.5 to 12; about 1 to 35% by weight of a drug or a food component; about 3 to 30% by weight of a disintegrant; and about 3 to 30% by weight of an excipient. Especially preferred is a film composition comprising, relative to the total weight of the film composition, about 60 to 80% by weight of a resin composition that is a copolymer obtained by copolymerizing partially hydrolyzed polyvinyl alcohol having an average degree of polymerization of about 300 to 500, methyl methacrylate, and acrylic acid at a weight ratio of about 60 to 90:7 to 38:0.5 to 12; about 5 to 30% by weight of a drug or a food component; about 5 to 20% by weight of a disintegrant; and about 5 to 20% by weight of an excipient.

More preferable embodiments of the film composition of the present invention are as follows. In particular, more preferred is a film composition comprising, relative to the total weight of the composition, about 1 to 90% by weight of a resin that is a copolymer obtained by copolymerizing partially hydrolyzed polyvinyl alcohol having an average degree of polymerization of about 300 to 500, methyl methacrylate, and acrylic acid at a weight ratio of about 60 to 90:7 to 38:0.5 to 12; about 0.01 to 50% by weight of a drug or a food component; about 1 to 50% by weight of crospovidone; and about 1 to 50% by weight of maltitol syrup. Further preferred is a film composition comprising, relative to the total weight of the composition, about 20 to 87.5% by weight of a resin composition that is a copolymer obtained by copolymerizing partially hydrolyzed polyvinyl alcohol having an average degree of polymerization of about 300 to 500, methyl methacrylate, and acrylic acid at a weight ratio of about 60 to 90:7 to 38:0.5 to 12; about 0.1 to 40% by weight of a drug or a food component; about 2 to 40% by weight of crospovidone; and about 2 to 40% by weight of maltitol syrup. Especially preferred is a film composition comprising, relative to the total weight of the composition, about 40 to 85% by weight of a resin that is a copolymer obtained by copolymerizing partially hydrolyzed polyvinyl alcohol having an average degree of polymerization of about 300 to 500, methyl methacrylate, and acrylic acid at a weight ratio of about 60 to 90:7 to 38:0.5 to 12; about 1 to 35% by weight of a drug or a food component; about 3 to 30% by weight of crospovidone; and about 3 to 30% by weight of maltitol syrup. Most preferred is a film composition comprising, relative to the total weight of the composition, about 60 to 80% by weight of a resin that is a copolymer obtained by copolymerizing partially hydrolyzed polyvinyl alcohol having an average degree of polymerization of about 300 to 500, methyl methacrylate, and acrylic acid at a weight ratio of about 60 to 90:7 to 38:0.5 to 12; about 5 to 30% by weight of a drug or a food component; about 5 to 20% by weight of crospovidone; and about 5 to 20% by weight of maltitol syrup.

Other preferable embodiments of the film composition of the present invention are as follows. In particular, preferred is a film composition comprising, relative to the total weight of the film composition, about 1 to 90% by weight of a resin that is a copolymer obtained by copolymerizing partially hydrolyzed polyvinyl alcohol having an average degree of polymerization of about 300 to 500, methyl methacrylate, and acrylic acid at a weight ratio of about 60 to 90:7 to 38:0.5 to 12; about 0.01 to 50% by weight of a drug or a food component; about 1 to 50% by weight of a disintegrant; about 1 to 50% by weight of an excipient; and about 1 to 50% by weight of a binder. More preferred is a film composition comprising, relative to the total weight of the film composition, about 20 to 87.5% by weight of a resin composition that is a copolymer obtained by copolymerizing partially hydrolyzed polyvinyl alcohol having an average degree of polymerization of about 300 to 500, methyl methacrylate, and acrylic acid at a weight ratio of about 60 to 90:7 to 38:0.5 to 12; about 0.1 to 40% by weight of a drug or a food component; about 2 to 40% by weight of a disintegrant; about 2 to 40% by weight of an excipient; and about 2 to 40% by weight of a binder. Further preferred is a film composition comprising, relative to the total weight of the film composition, about 40 to 85% by weight of a resin composition that is a copolymer obtained by copolymerizing partially hydrolyzed polyvinyl alcohol having an average degree of polymerization of about 300 to 500, methyl methacrylate, and acrylic acid at a weight ratio of about 60 to 90:7 to 38:0.5 to 12; about 1 to 35% by weight of a drug or a food component; about 3 to 30% by weight of a disintegrant; about 3 to 30% by weight of an excipient; and about 3 to 30% by weight of a binder. Especially preferred is a film composition comprising, relative to the total weight of the film composition, about 60 to 80% by weight of a resin composition that is a copolymer obtained by copolymerizing partially hydrolyzed polyvinyl alcohol having an average degree of polymerization of about 300 to 500, methyl methacrylate, and acrylic acid at a weight ratio of about 60 to 90:7 to 38:0.5 to 12; about 5 to 30% by weight of a drug or a food component; about 5 to 20% by weight of a disintegrant; about 5 to 20% by weight of an excipient; and about 5 to 20% by weight of a binder.

The thickness of the film composition of the present invention can generally be about 0.1 to 1000 µm, preferably about 10 to 200 µm. In cases where the film composition is an orally administered drug, the film composition is in such a size as to easily put in the mouth. In cases where the film composition is an externally applied drug, the film composition is in an appropriate size to attach to an affected site.

The film composition of the present invention may be either orally administered or dermally administered.

In cases where the film composition is orally administered, the film composition may be taken with water, and more preferably the film composition rapidly dissolves and disintegrates in saliva and the like in the mouth without the need for water. As a pharmaceutical preparation that rapidly dissolves and disintegrates in the mouth, an orally disintegrating tablet is generally used. Compared with the orally disintegrating tablet, the drug-containing film of the present invention is easy to carry and the production process thereof is not complicated. The drug-containing film of the present invention generally dissolves and disintegrates in the mouth in about 60 seconds or less, preferably about 45 seconds or less, more preferably about 30 seconds or less, especially preferably about 20 seconds or less.

In cases where the film composition is dermally administered, the surface of the film composition can be moistened with a small amount of water and attached to the skin and the like.

The film composition of the present invention has a convenient strength for ordinary use, and the strength is, for example, about 80 N/mm or more, preferably about 85 N/mm or more, more preferably about 90 N/mm or more.

The film composition of the present invention can be produced according to a production process of a common drug-containing film or food-containing film. For example, the film composition can be produced by a process comprising (1) a step of dissolving or dispersing the polyvinyl alcohol copolymer mentioned above and either the drug or the food component in a mixed solution of water and an organic solvent to give a solution or dispersion solution; and
(2) a step of spreading the obtained solution or dispersion solution into a mold of a film, removing the solvent with drying to give a film, peeling the film off the mold, and cutting the film into a desired size.

In the step (1), it is preferable to first dissolve or suspend the polyvinyl alcohol copolymer and either the drug or the food component, and as needed a sugar, a disintegrant, a binder, and any other additive in water, and then to add an organic solvent. The organic solvent is not specifically limited as long as the organic solvent dissolves each component. Specific examples of the organic solvent include an alcohol (for example, ethanol and methanol), acetone, and methylene chloride. Such an organic solvent may be used alone or in combination of two or more. Among these, ethanol is preferable. In cases where bubbles are formed in the solution during the preparation of the film in the step (1), it is advisable to leave the solution overnight or to vacuum degas the solution.

The viscosity of the solution in which the polyvinyl alcohol copolymer is dissolved in the step (1) means a viscosity determined at a rotational speed of 60 rpm and at a temperature of 25°C according to the method for determining a viscosity (JIS K 7117-2) specified in Japanese Industrial Standards (JIS). The viscosity is generally about 10 to 400 mPa·s, preferably about 10 to 250 mPa·s, more preferably about 10 to 200 mPa·s. The viscosity can be determined with a marketed viscometer, for example, a B type viscometer (manufactured by TOKYO KEIKI INC.). When the viscosity of the solution is too high, the film composition might not be formed efficiently.

The drying of the solvent in the step (2) can be appropriately carried out at a temperature of generally about 20 to 60°C, preferably about 20 to 50°C. As the drying method, air drying is preferable because the film is hardly damaged. The film thus formed is cut into an appropriate size and used as the film composition.

### EXAMPLES

The present invention will be described below with reference to the following Examples and Comparative Examples, but the present invention is not limited thereto.

### EXAMPLE 1

### (1) Production of a resin

A resin was produced by a method described in WO 2005/019286. That is, 175.8 g of polyvinyl alcohol (the polymerization degree of 500, the hydrolysis degree of 88 mol%, manufactured by NIPPON GOHSEI Co., Ltd.) and 582.3 g of deionized water were placed in a separable flask equipped with a reflux condenser, a dropping funnel, a thermometer, a nitrogen inlet, and a stirrer. The polyvinyl alcohol was dispersed in the water at normal temperature and completely dissolved at 95°C. Next, 5.4 g of acrylic acid and 37.3 g of methyl methacrylate were added thereto (the polyvinyl alcohol: the polymerizable vinyl monomers (wt%) = 102:25). The air in the flask was replaced with a nitrogen gas, and the mixture was heated up to 50°C. To the mixture, 8.5 g of tertiary butyl hydroperoxide and 8.5 g of sodium erythorbate were added. The mixture was reacted for four hours to give a polyvinyl alcohol copolymer. The polyvinyl alcohol copolymer was dried and crushed by a usual method to give a polyvinyl alcohol copolymer powder.

### (2) Production of a drug-containing film

The polyvinyl alcohol copolymer obtained in the above (1) (hereinafter sometimes abbreviated to the PVA copolymer), isopropylantipyrine (IPA), maltitol syrup (brand name: LESYS, registered trademark, manufactured by Towa Kasei Industry Co.,Ltd.), and crospovidone (manufactured by BASF Japan Ltd.) in the amounts shown in Table 1 were gradually added into purified water with stirring to prepare a 20% (w/w) aqueous solution of the polyvinyl alcohol copolymer. To the solution, ethanol and purified water were added with stirring so that the concentration of the ethanol was 43.9% (w/w). Finally, a 5.3% (w/w) aqueous solution of the polyvinyl alcohol copolymer was obtained.

The obtained solution was spread into a mold of a film, and the solvent of the solution was removed with drying at room temperature to give a film. The film thus formed was peeled off the mold, and cut into 10 mm × 30 mm to give drug-containing films. The thickness of the obtained drug-containing film (hereinafter referred to as a present invention 1) was about 120 µm.

**Table 1**

| Component | Amount (% by weight) |
|---|---|
| PVA copolymer | 60 |
| Crospovidone | 20 |
| Maltitol syrup | 10 |
| IPA | 10 |
| Total | 100 |

### EXAMPLE 2

A drug-containing film was produced in the same manner as in Example 1 except that the amount of the maltitol syrup was 15% by weight and the amount of the IPA was 5% by weight (hereinafter referred to as a present invention 2).

### EXAMPLE 3

A drug-containing film was produced in the same manner as in Example 1 except that the amount of the maltitol syrup was 19% by weight and the amount of the IPA was 1% by weight (hereinafter referred to as a present invention 3).

### EXAMPLE 4

A drug-containing film was produced in the same manner as in Example 1 except that the amount of each component was as shown in Table 2 below (hereinafter referred to as a present invention 4).

**Table 2**

| Component | Amount (% by weight) |
|---|---|
| PVA copolymer | 80 |
| Crospovidone | 10 |
| Maltitol syrup | 9 |
| IPA | 1 |
| Total | 100 |

### COMPARATIVE EXAMPLE 1

A drug-containing film was obtained in the same manner as in Example 1 except that the components and their amounts were as shown in Table 3 below, i.e., instead of the PVA copolymer, hydroxypropyl methyl cellulose (manufactured by Shin-Etsu Chemical Co., Ltd.) was used (referred to as a comparative product 1).

**Table 3**

| Component | Amount (% by weight) |
|---|---|
| Hydroxypropyl methyl cellulose (HPMC) | 60 |
| Crospovidone | 20 |
| Maltitol syrup | 10 |
| IPA | 10 |
| Total | 100 |

### COMPARATIVE EXAMPLE 2

A drug-containing film was obtained in the same manner as in Comparative Example 1 except that the amount of the maltitol syrup was 15% by weight and the amount of the IPA was 5% by weight (referred to as a comparative product 2).

### COMPARATIVE EXAMPLE 3

A drug-containing film was obtained in the same manner as in Comparative Example 1 except that the amount of the maltitol syrup was 19% by weight and the amount of the IPA was 1% by weight (referred to as a comparative product 3).

### COMPARATIVE EXAMPLE 4

A drug-containing film was produced in the same manner as in Example 1 except that the amount of each component was as shown in Table 4 below (hereinafter referred to as a comparative product 4).

**Table 4**

| Component | Amount (% by weight) |
|---|---|
| Hydroxypropyl methyl cellulose (HPMC) | 80 |
| Crospovidone | 10 |
| Maltitol syrup | 9 |
| IPA | 1 |
| Total | 100 |

### COMPARATIVE EXAMPLE 5

A drug-containing film was obtained in the same manner as in Comparative Example 3 except that instead of the hydroxymethyl cellulose, polyvinyl alcohol (the polymerization degree of 500, the hydrolysis degree of 88 mol%, manufactured by NIPPON GOHSEI Co., Ltd.) was used (hereinafter referred as a comparative product 5).

### TEST EXAMPLE

The present inventions 1 to 4 obtained in Examples 1 to 4 and the comparative products 1 to 5 obtained in Comparative Examples 1 to 5 were evaluated in terms of the capability of forming a film, the film strength, and the dissolution time in the mouth.

The capability of forming a film was judged according to the following evaluation criteria.

Evaluation criteria of the capability of forming a film Very good: A transparent film is formed.

Good: A film is formed although the film partly opacifies. Average: A film is formed although the film totally opacifies. Poor: A film is not formed.

The film strength was determined from the maximum load when the film was pulled at a testing speed of 5 mm/min with the use of a digital force gauge FGS-50TV (manufactured by Nidec-Shimpo Corporation). The dissolution time in the mouth was measured as the time elapsed from the moment the film was put in the mouth until the film completely dissolved.

### Results

The results are shown in Table 5. In addition, when the polyvinyl alcohol copolymer or hydroxypropyl methyl cellulose was used alone without any drugs or food components or additives, film formation was achieved.

**Table 5**

| Film | Amount of drug (% by weight) | Capability of forming film | Film strength (N/mm) | Dissolution time in the mouth (seconds) |
|---|---|---|---|---|
| Present invention 1 | 10 | Good | 102 | 15 |
| Present invention 2 | 5 | Very good | 123 | 13 |
| Present invention 3 | 1 | Very good | 121 | 15 |
| Present invention 4 | 1 | Very good | 229 | 30 |
| Comparative product 1 | 10 | Poor | - | - |
| Comparative product 2 | 5 | Poor | - | - |
| Comparative product 3 | 1 | Average | 104 | 38 |
| Comparative product 4 | 1 | Very good | 213 | 68 |
| Comparative product 5 | 1 | Poor | - | - |

From the above results, it was confirmed that when a polyvinyl alcohol copolymer is used as a base of the film composition, the film composition is easily formed and the film composition has an excellent strength and a short dissolution time in the mouth. In particular, the composition comprising the polyvinyl alcohol copolymer was easily formed into a film and rapidly dissolved in the mouth compared with the composition comprising hydroxypropyl methyl cellulose, which is conventionally used as a base of a film coating or a drug-containing film. It was therefore confirmed that the film composition of the present invention can be prepared as a rapidly dissolving film composition containing a large amount of a drug or a food component. The film composition can also contain a large amount of additives besides the drug or the food component, and thus the film composition can be given various functions. For example, by adding a sweetener and the like, a film composition having a lowered bitter taste can be prepared.

### INDUSTRIAL APPLICABILITY

According to the present invention, a film composition that is easily formed can be obtained using, as a base, a resin obtained by copolymerizing polyvinyl alcohol and at least one or more polymerizable vinyl monomers.

## Claims

1. A film composition comprising
(i) a resin obtained by copolymerizing polyvinyl alcohol and at least one or more polymerizable vinyl monomers; and
(ii) a drug or a food component.

2. The composition according to claim 1, wherein the composition is a drug-containing film comprising the resin obtained by copolymerizing the polyvinyl alcohol and the at least one or more polymerizable vinyl monomers; and the drug.

3. The composition according to claim 1, wherein the composition is a food-containing film comprising the resin obtained by copolymerizing the polyvinyl alcohol and the at least one or more polymerizable vinyl monomers; and the food component.

4. The composition according to claim 1, wherein the polyvinyl alcohol and the polymerizable vinyl monomer are copolymerized at a weight ratio of 6:4 to 9:1.

5. The composition according to claim 1, wherein the polyvinyl alcohol has an average degree of polymerization of 1300 or less.

6. The composition according to claim 5, wherein the polyvinyl alcohol has an average degree of polymerization of 200 to 1300.

7. The composition according to claim 5, wherein the polyvinyl alcohol has an average degree of polymerization of 200 to 900.

8. The composition according to claim 5, wherein the polyvinyl alcohol has an average degree of polymerization of 300 to 500.

9. The composition according to claim 1, wherein the polyvinyl alcohol is partially hydrolyzed polyvinyl alcohol.

10. The composition according to claim 1, wherein the polymerizable vinyl monomer is at least one or more selected from the group consisting of acrylic acid, methacrylic acid, methyl acrylate, ethyl acrylate, butyl acrylate, isobutyl acrylate, methyl methacrylate, ethyl methacrylate, butyl methacrylate, and isobutyl methacrylate.

11. The composition according to claim 1, wherein the resin is a copolymer of partially hydrolyzed polyvinyl alcohol having an average degree of polymerization of 300 to 500, methyl methacrylate, and acrylic acid.

12. The composition according to claim 11, wherein the resin is a copolymer obtained by copolymerizing partially hydrolyzed polyvinyl alcohol having an average degree of polymerization of 300 to 500, methyl methacrylate, and acrylic acid at a weight ratio of 60 to 90:7 to 38:0.5 to 12.

13. The composition according to claim 1, wherein the amount of the resin is 1 to 90% by weight relative to the total weight of the composition.

14. The composition according to claim 13, wherein the amount of the resin is 60 to 80% by weight relative to the total weight of the composition.

15. The composition according to claim 1, wherein the amount of the drug or the food component is 0.01 to 50% by weight relative to the total weight of the composition.

16. The composition according to claim 15, wherein the amount of the drug or the food component is 5 to 30% by weight relative to the total weight of the composition.

17. The composition according to claim 1, wherein the composition further comprises at least one or more selected from the group consisting of a disintegrant, an excipient, and a binder.

18. The composition according to claim 17, wherein the disintegrant is at least one or more selected from the group consisting of crospovidone, starch, carmellose, carmellose calcium, carboxy starch sodium, carboxymethyl starch sodium, low-substituted hydroxypropyl cellulose, guar gum, croscarmellose sodium, pregelatinized starch, agar powder, and gum arabic.

19. The composition according to claim 18, wherein the disintegrant is crospovidone.

20. The composition according to claim 18, wherein the amount of the disintegrant is 1 to 50% by weight relative to the total weight of the composition.

21. The composition according to claim 20, wherein the amount of the disintegrant is 5 to 20% by weight relative to the total weight of the composition.

22. The composition according to claim 17, wherein the excipient is at least one or more selected from the group consisting of maltitol syrup, crystalline cellulose, erythritol, D-sorbitol, xylitol, sorbitol, trehalose, D-mannitol, maltitol, mannitol, glucose, fructose, maltose, sucrose, starch syrup, saccharose, glucose, lactose, anhydrous silicic acid, dibasic calcium phosphate, anhydrous dibasic calcium phosphate, calcium silicate, hydrous silicon dioxide, magnesium aluminometasilicate, synthetic hydrotalcite, calcium carbonate, precipitated calcium carbonate, magnesium carbonate, corn starch, potato starch, wheat starch, rice starch, and partially pregelatinized starch.

23. The composition according to claim 22, wherein the excipient is maltitol syrup.

24. The composition according to claim 22, wherein the amount of the excipient is 1 to 50% by weight relative to the total weight of the composition.

25. The composition according to claim 24, wherein the amount of the excipient is 5 to 20% by weight relative to the total weight of the composition.

26. The composition according to claim 17, wherein the binder is at least one or more selected from the group consisting of hydroxypropyl methyl cellulose, hydroxypropyl cellulose, polyvinylpyrrolidone, methyl cellulose, polyvinyl acetal diethylamino acetate, a hydroxypropyl methyl cellulose mixture, carmellose, carmellose sodium, carboxy starch sodium, croscarmellose sodium, crospovidone, hydroxyethyl methyl cellulose, hydroxypropyl starch, hydroxyethyl starch, crystalline cellulose, pullulan, gum arabic, and sodium alginate.

27. The composition according to claim 17, wherein the composition comprises, relative to the total weight of the composition, 1 to 90% by weight of the resin that is a copolymer obtained by copolymerizing partially hydrolyzed polyvinyl alcohol having an average degree of polymerization of 300 to 500, methyl methacrylate, and acrylic acid at a weight ratio of 60 to 90:7 to 38:0.5 to 12; 0.01 to 50% by weight of the drug or the food component; 1 to 50% by weight of the disintegrant; and 1 to 50% by weight of the excipient.

28. The composition according to claim 27, wherein the composition comprises, relative to the total weight of the composition, 60 to 80% by weight of the resin that is a copolymer obtained by copolymerizing partially hydrolyzed polyvinyl alcohol having an average degree of polymerization of 300 to 500, methyl methacrylate, and acrylic acid at a weight ratio of 60 to 90:7 to 38:0.5 to 12; 5 to 30% by weight of the drug or the food component; 5 to 20% by weight of the disintegrant; and 5 to 20% by weight of the excipient.
